(19) Europäisches Patentamt / European Patent Office / Office européen des brevets

(11) EP 1 929 868 B1

(12) **EUROPÄISCHE PATENTSCHRIFT**

(45) Veröffentlichungstag und Bekanntmachung des Hinweises auf die Patenterteilung:
**25.01.2017 Patentblatt 2017/04**

(51) Int Cl.:
***A01N 47/24*** (2006.01) ***A01N 43/653*** (2006.01)
***A01P 3/00*** (2006.01)

(21) Anmeldenummer: **08153111.3**

(22) Anmeldetag: **26.02.2003**

(54) **Fungizide Mischungen auf der Basis von Prothioconazole und einem Strobilurinderivat**

Fungicidal mixtures based on prothioconazole and a strobilurin derivative

Mélanges fongicides à base de prothioconazole et d'un dérivé de strobilurine

(84) Benannte Vertragsstaaten:
**AT BE BG CH CY CZ DE DK EE ES FI FR GB GR HU IE IT LI LU MC NL PT SE SI SK TR**
Benannte Erstreckungsstaaten:
**LT LV RO**

(30) Priorität: **01.03.2002 DE 10208838**

(43) Veröffentlichungstag der Anmeldung:
**11.06.2008 Patentblatt 2008/24**

(62) Dokumentnummer(n) der früheren Anmeldung(en) nach Art. 76 EPÜ:
**05026582.6 / 1 642 499**
**03743328.1 / 1 482 798**

(73) Patentinhaber: **BASF SE**
**67056 Ludwigshafen am Rhein (DE)**

(72) Erfinder:
- **Ammermann, Eberhard**
  **64646, Heppenheim (DE)**
- **Stierl, Reinhard**
  **83345, Kaohsiung County (TW)**
- **Lorenz, Gisela**
  **67434, Neustadt (DE)**
- **Strathmann, Siegfried**
  **67117, Limburgerhof (DE)**
- **Schelberger, Klaus**
  **67161, Gönnheim (DE)**
- **Spadafora, V. James**
  **Sugar Land, TX 77478 (US)**
- **Christen, Thomas**
  **67125, Dannstadt (DE)**

(74) Vertreter: **BASF IP Association**
**BASF SE**
**ZRX-C6**
**67056 Ludwigshafen (DE)**

(56) Entgegenhaltungen:
**WO-A-97/40688** **WO-A-98/47367**
**WO-A-02/056686**

- **JAYASENA K W ET AL: "Evaluation of fungicides in control of spot-type net blotch on barley", CROP PROTECTION, ELSEVIER SCIENCE, GB, Bd. 21, Nr. 1, 1. Februar 2002 (2002-02-01), Seiten 63-69, XP002347515, ISSN: 0261-2194, DOI: 10.1016/S0261-2194(01)00118-1**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 929 868 B1

## Beschreibung

Die Erfindung betrifft eine fungizide Mischung gemäß Anspruch 1, ein Verfahren zur Bekämpfung von Schadpilzen gemäß Anspruch 2 und ein fungizides Mittel gemäß Anspruch 5.

Die Verbindung der Formel I, das 2-[2-(1-Chlorcyclopropyl)-3-(2-chlorphenyl)-2-hydroxypropyl]-2,4-dihydro-[1,2,4]-triazol-3-thion (Prothioconazol) ist bereits aus der WO 96/16048 bekannt.

Aus der WO 98/47367 ist eine Reihe von Wirkstoffkombinationen von Prothioconazol mit einer Vielzahl anderer fungizider Verbindungen bekannt.

Auch das Strobilurin-Derivat der Formel IV Pyraclostrobin ist bereits bekannt und in der EP-A-0 804 421 beschrieben.

Im Hinblick auf eine Senkung der Aufwandmengen und eine Verbesserung des Wirkungsspektrums der bekannten Verbindungen I, IV, lagen der vorliegenden Erfindung Mischungen als Aufgabe zugrunde, die bei verringerter Gesamtmenge an ausgebrachten Wirkstoffen eine verbesserte Wirkung gegen Schadpilze aufweisen (synergistische Mischungen).

Demgemäß wurde die eingangs definierte Mischung von Prothioconazol mit mindestens einem Strobilurin-Derivat gefunden. Es wurde außerdem gefunden, daß sich bei gleichzeitiger, und zwar gemeinsamer oder getrennter Anwendung der Verbindung I und der Verbindung IV oder der Verbindung I und der Verbindung IV nacheinander Schadpilze besser bekämpfen lassen, als mit den Einzelverbindungen allein.

Das 2-[2-(1-Chlorcyclopropyl)-3-(2-chlorphenyl)-2-hydroxypropyl]-2,4-dihydro-[1,2,4]-triazol-3-thion der Formel I ist aus der WO 96-16 048 bekannt. Die Verbindung kann in der "Thiono"-Form der Formel

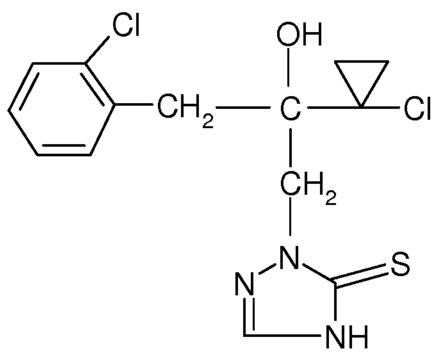

(I)

oder in der tautomeren "Mercapto"-Form der Formel

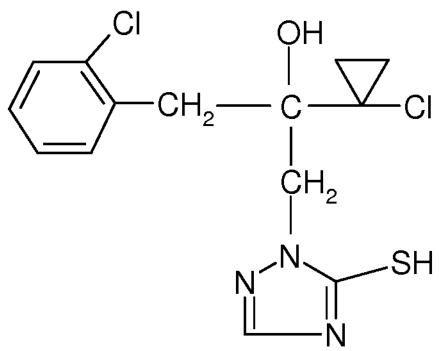

(Ia)

vorliegen. Der Einfachheit halber wird jeweils nur die "Thiono"-Form aufgeführt.

Pyraclostrobin der Formel IV

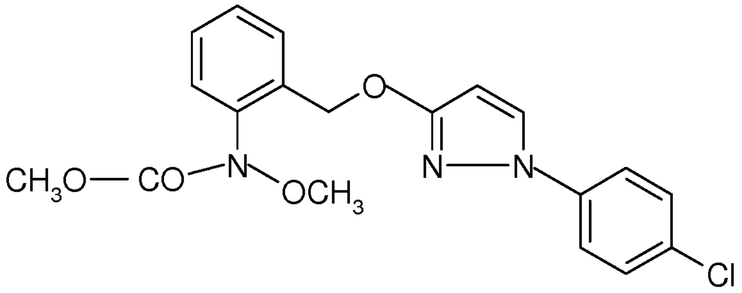

(IV) (Pyraclostrobin)

ist aus der EP-A 0 804 421 bekannt.

Die Verbindungen I und IV sind wegen des basischen Charakters der in ihnen enthaltenen Stickstoffatome in der Lage, mit anorganischen oder organischen Säuren oder mit Metallionen Salze oder Addukte zu bilden.

Beispiele für anorganische Säuren sind Halogenwasserstoffsäuren wie Fluorwasserstoff, Chlorwasserstoff, Bromwasserstoff und Jodwasserstoff, Schwefelsäure, Phosphorsäure und Salpetersäure.

Als organischen Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxalsäure, Alkylsulfonsäuren (Sulfonsäuren mit geradkettigen oder verzweigten Alkylresten mit 1 bis 20 Kohlenstoffatomen), Arylsulfonsäuren oder - disulfonsäuren (aromatische Reste wie Phenyl und Naphthyl welche eine oder zwei Sulfonsäuregruppen tragen), Alkylphosphonsäuren (Phosphonsäuren mit geradkettigen oder verzweigten Alkylresten mit 1 bis 20 Kohlenstoffatomen), Arylphosphonsäuren oder -diphosphonsäuren (aromatische Reste wie Phenyl und Naphthyl welche eine oder zwei Phosphorsäurereste tragen), wobei die Alkyl- bzw. Arylreste weitere Substituenten tragen können, z.B. p-Toluolsulfonsäure, Salizylsäure, p-Aminosalizylsäure, 2-Phenoxybenzoesäure, 2-Acetoxybenzoesäure etc.

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der Nebengruppen der vierten Periode. Die Metalle können dabei in den verschiedenen ihnen zukommenden Wertigkeiten vorliegen.

Bevorzugt sind Mischungen von Prothioconazole mit Pyraclostrobin der Formel IV.

Bevorzugt setzt man bei der Bereitstellung der Mischungen die reinen Wirkstoffe I und IV ein, denen man weitere Wirkstoffe gegen Schadpilze oder gegen andere Schädlinge wie Insekten, Spinntiere oder Nematoden oder auch herbizide oder wachstumsregulierende Wirkstoffe oder Düngemittel beimischen kann.

Die Mischungen aus der Verbindung I mit der Verbindung IV bzw. die Verbindung I mit der Verbindung IV gleichzeitig, gemeinsam oder getrennt angewandt, zeichnen sich durch eine hervorragende Wirkung gegen ein breites Spektrum von pflanzenpathogenen Pilzen, insbesondere aus der Klasse der Ascomyceten, Basidiomyceten, Phycomyceten und Deuteromyceten aus. Sie sind z.T. systemisch wirksam und können daher auch als Blatt- und Bodenfungizide eingesetzt werden.

Besondere Bedeutung haben sie für die Bekämpfung einer Vielzahl von Pilzen an verschiedenen Kulturpflanzen wie Baumwolle, Gemüsepflanzen (z.B. Gurken, Bohnen, Tomaten, Kartoffeln und Kürbisgewächse), Gerste, Gras, Hafer, Bananen, Kaffee, Mais, Obstpflanzen, Reis, Roggen, Soja, Wein, Weizen, Zierpflanzen, Zuckerrohr sowie an einer Vielzahl von Samen.

Insbesondere eignen sie sich zur Bekämpfung der folgenden pflanzenpathogenen Pilze: Blumeria graminis (echter Mehltau) an Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle, Reis und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinera (Grauschimmel) an Erdbeeren, Gemüse, Zierpflanzen und Reben, Cercospora arachidicola an Erdnüssen, Pseudocercosporella herpotrichoides an Weizen und Gerste, Pyricularia oryzae an Reis, Phytophthora infestans an Kartoffeln und Tomaten, Plasmopara viticola an Reben, Pseudoperonospora-Arten in Hopfen und Gurken, Alternaria-Arten an Gemüse und Obst, Mycosphaerella-Arten in Bananen sowie Fusarium- und Verticillium-Arten.

Sie sind außerdem im Materialschutz (z.B. Holzschutz) anwendbar, beispielsweise gegen Paecilomyces variotii.

Die Verbindung I mit der Verbindung IV können gleichzeitig, und zwar gemeinsam oder getrennt, oder nacheinander aufgebracht werden, wobei die Reihenfolge bei getrennter Applikation im allgemeinen keine Auswirkung auf den Bekämpfungserfolg hat.

Die Verbindungen I und IV werden üblicherweise in einem Gewichtsverhältnis von 20:1 bis 1:20, insbesondere 10:1 bis 1:10, vorzugsweise 5:1 bis 1:5 angewendet.

Die Aufwandmengen der erfindungsgemäßen Mischungen liegen, vor allem bei landwirtschaftlichen Kulturflächen, je nach Art des gewünschten Effekts bei 0,01 bis 8 kg/ha, vorzugsweise 0,1 bis 5 kg/ha, insbesondere 0,1 bis 3,0 kg/ha.

Die Aufwandmengen liegen dabei für die Verbindung I bei 0,01 bis 1 kg/ha, vorzugsweise 0,05 bis 0,5 kg/ha, insbesondere 0,05 bis 0,3 kg/ha.

Die Aufwandmengen für die Verbindung IV liegen entsprechend bei 0,01 bis 1 kg/ha, vorzugsweise 0,02 bis 0,5 kg/ha, insbesondere 0,05 bis 0,3 kg/ha.

Bei der Saatgutbehandlung werden im allgemeinen Aufwandmengen an Mischung von 0,001 bis 250 g/kg Saatgut, vorzugsweise 0,01 bis 100 g/kg, insbesondere 0,01 bis 50 g/kg verwendet.

Sofern für Pflanzen pathogene Schadpilze zu bekämpfen sind, erfolgt die getrennte oder gemeinsame Applikation der Verbindung I mit der Verbindung IV oder der Mischungen aus der Verbindung I mit der Verbindung IV durch Besprühen oder Bestäuben der Samen, der Pflanzen oder der Böden vor oder nach der Aussaat der Pflanzen oder vor oder nach dem Auflaufen der Pflanzen.

Die erfindungsgemäßen fungiziden synergistischen Mischungen bzw. die Verbindung I und der Verbindung IV können beispielsweise in Form von direkt versprühbaren Lösungen, Pulver und Suspensionen oder in Form von hochprozentigen wäßrigen, öligen oder sonstigen Suspensionen, Dispersionen, Emulsionen, Öldispersionen, Pasten, Stäubemitteln, Streumitteln oder Granulaten aufbereitet und durch Versprühen, Vernebeln, Verstäuben, Verstreuen oder Gießen angewendet werden. Die Anwendungsform ist abhängig vom Verwendungszweck; sie soll in jedem Fall eine möglichst feine und gleichmäßige Verteilung der erfindungsgemäßen Mischung gewährleisten.

Die Formulierungen werden in an sich bekannter Weise hergestellt, z.B. durch Zugabe von Lösungsmitteln und/oder Trägerstoffen. Den Formulierungen werden üblicherweise inerte Zusatzstoffe wie Emulgiermittel oder Dispergiermittel beigemischt.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanole oder Fettalkoholglycolethern, Kondensationsprodukte von sulfoniertem Naphthalin und seinen Derivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol- oder Tributylphenylpolyglycolether, Alkylarylpolyetheralkohole, Isotridecylalkohol, Fettalkoholethylenoxid- Kondensate, ethoxyliertes Rizinusöl, Polyoxyethylenalkylether oder Polyoxypropylen, Laurylalkoholpolyglycoletheracetat, Sorbitester, Lignin-Sulfitablaugen oder Methylcellulose in Betracht.

Pulver Streu- und Stäubemittel können durch Mischen oder gemeinsames Vermahlen der Verbindung I und der Verbindung IV oder der Mischung aus den Verbindungen I und IV mit einem festen Trägerstoff hergestellt werden.

Granulate (z.B. Umhüllungs-, Imprägnierungs- oder Homogengranulate) werden üblicherweise durch Bindung des Wirkstoffs oder der Wirkstoffe an einen festen Trägerstoff hergestellt.

Als Füllstoffe bzw. feste Trägerstoffe dienen beispielsweise Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, sowie Düngemittel wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Formulierungen enthalten im allgemeinen 0,1 bis 95 Gew.-%, vorzugsweise 0,5 bis 90 Gew.-% der Verbindung I und der Verbindung IV bzw. der Mischung aus der Verbindung I mit der Verbindung IV. Die Wirkstoffe werden dabei in einer Reinheit von 90% bis 100%, vorzugsweise 95% bis 100% (nach NMR- oder HPLC-Spektrum) eingesetzt.

Die Anwendung der Verbindung I und der Verbindung IV oder der Mischungen oder der entsprechenden Formulierungen erfolgt so, daß man die Schadpilze, deren Lebensraum oder die von ihnen freizuhaltenden Pflanzen, Samen, Böden, Flächen, Materialien oder Räume mit einer fungizid wirksamen Menge der Mischung, bzw. der Verbindung I und der Verbindung IV bei getrennter Ausbringung, behandelt.

Die Anwendung kann vor oder nach dem Befall durch die Schadpilze erfolgen.

Anwendungsbeispiel

Die synergistische Wirkung der erfindungsgemäßen Mischungen ließ sich durch die folgenden Versuche zeigen:

Die Wirkstoffe wurden getrennt oder gemeinsam als 10%ige Emulsion in einem Gemisch aus 63 Gew.-% Cyclohexanon und 27 Gew.-% Emulgator aufbereitet und entsprechend der gewünschten Konzentration mit Wasser verdünnt.

Die Auswertung erfolgte durch Feststellung der befallenen Blattflächen in Prozent. Diese Prozent-Werte wurden in Wirkungsgrade umgerechnet. Der Wirkungsgrad (W) wurde nach der Formel von Abbot wie folgt bestimmt:

$$W = (1 - \alpha/\beta) \cdot 100$$

α entspricht dem Pilzbefall der behandelten Pflanzen in % und
β entspricht dem Pilzbefall der unbehandelten (Kontroll-) Pflanzen in %

Bei einem Wirkungsgrad von 0 entspricht der Befall der behandelten Pflanzen demjenigen der unbehandelten Kontrollpflanzen; bei einem Wirkungsgrad von 100 wiesen die behandelten Pflanzen keinen Befall auf.

Die zu erwartenden Wirkungsgrade der Wirkstoffmischungen wurden nach der Colby Formel [R.S. Colby, Weeds 15, 20-22 (1967)] ermittelt und mit den beobachteten Wirkungsgraden verglichen.

Colby Formel: $E = x + y - x \cdot y/100$

E zu erwartender Wirkungsgrad, ausgedrückt in % der unbehandelten Kontrolle, beim Einsatz der Mischung aus den Wirkstoffen A und B in den Konzentrationen a und b
x der Wirkungsgrad, ausgedrückt in % der unbehandelten Kontrolle, beim Einsatz des Wirkstoffs A in der Konzentration a
y der Wirkungsgrad, ausgedrückt in % der unbehandelten Kontrolle, beim Einsatz des Wirkstoffs B in der Konzentration b

Anwendungsbeispiel 1: Wirksamkeit gegen Weizenmehltau verursacht durch *Erysiphe [syn. Blumeria] graminis* forma specialis. *tritici*

Blätter von in Töpfen gewachsenen Weizenkeimlingen der Sorte "Kanzler" wurden mit wäßriger Wirkstoffaufbereitung, die aus einer Stammlösung bestehend aus 10 % Wirkstoff, 85 % Cyclohexanon und 5 % Emulgiermittel angesetzt wurde, bis zur Tropfnässe besprüht und 24 Stunden nach dem Antrocknen des Spritzbelages mit Sporen des Weizenmehltaus (*Erysiphe [syn. Blumeria] graminis* forma specialis. *tritici*) bestäubt. Die Versuchspflanzen wurden anschließend im Gewächshaus bei Temperaturen zwischen 20 und 24°C und 60 bis 90 % relativer Luftfeuchtigkeit aufgestellt. Nach 7 Tagen wurde das Ausmaß der Mehltauentwicklung visuell in % Befall der gesamten Blattfläche ermittelt.

Die visuell ermittelten Werte für den Prozentanteil befallener Blattflächen wurden in Wirkungsgrade als % der unbehandelten Kontrolle umgerechnet. Wirkungsgrad 0 ist gleicher Befall wie in der unbehandelten Kontrolle, Wirkungsgrad 100 ist 0 % Befall. Die zu erwartenden Wirkungsgrade für Wirkstoffkombinationen wurden nach der obengenannten Colby-Formel ermittelt und mit den beobachteten Wirkungsgraden verglichen.

Tabelle 1

| Wirkstoff | Wirkstoffkonzentration in der Spritzbrühe in ppm | Wirkungsgrad in % der unbehandelten Kontrolle |
|---|---|---|
| Kontrolle (unbehandelt) | (90 % Befall) | 0 |
| Verbindung I = Prothioconazol | 4<br>1<br>0.25<br>0,06<br>0,015 | 22<br>0<br>0<br>0<br>0 |
| Verbindung IV = Pyraclostrobin | 1<br>0.25 | 0<br>0 |

Tabelle 2

| Erfindungsgemäße Kombinationen | Beobachteter Wirkungsgrad | Berechneter Wirkungsgrad*) |
|---|---|---|
| Verbindung I = Prothioconazol + Verbindung IV = Pyraclostrobin 0,06+1 ppm Mischung 1 : 16 | 33 | 0 |

(fortgesetzt)

| Erfindungsgemäße Kombinationen | Beobachteter Wirkungsgrad | Berechneter Wirkungsgrad*) |
|---|---|---|
| Verbindung I = Prothioconazol + Verbindung IV = Pyraclostrobin 0,015+0,25 ppm Mischung 1 : 16 | 33 | 0 |
| Verbindung I = Prothioconazol + Verbindung IV = Pyraclostrobin 0,25 + 1 ppm Mischung 1 : 4 | 33 | 0 |
| Verbindung I = Prothioconazol + Verbindung IV = Pyraclostrobin 0,06 + 0,25 ppm Mischung 1 : 4 | 22 | 0 |
| Verbindung I = Prothioco-nazol + Verbindung IV = Pyraclostrobin 4 + 1 ppm Mischung 4 : 1 | 33 | 22 |
| *) berechnet nach der Colby-Formel | | |

Aus den Ergebnissen des Versuches geht hervor, daß der beobachteter Wirkungsgrad in allen Mischungsverhältnissen höher ist, als nach der Colby-Formel vorausberechnete Wirkungsgrad (aus Synerg 171. XLS).

Anwendungsbeispiel 2: Kurative Wirksamkeit gegen Weizenbraunrost verursacht durch *Puccinia recondita*

Blätter von in Töpfen gewachsenen Weizensämlingen der Sorte "Kanzler" wurden mit Sporen des Braunrostes (*Puccinia recondita*) bestäubt. Danach wurden die Töpfe für 24 Stunden in eine Kammer mit hoher Luftfeuchtigkeit (90 bis 95 %) und 20 bis 22_ C gestellt. Während dieser Zeit keimten die Sporen aus und die Keimschläuche drangen in das Blattgewebe ein. Die infizierten Pflanzen wurden am nächsten Tag mit einer wäßrigen Wirkstoffaufbereitung, die aus einer Stammlösung bestehend aus 10 % Wirkstoff, 85 % Cyclohexanon und 5 % Emulgiermittel angesetzt wurde, tropfnaß besprüht. Nach dem Antrocknen des Spritzbelages wurden die Versuchspflanzen im Gewächshaus bei Temperaturen zwischen 20 und 22_ C und 65 bis 70 % relativer Luftfeuchte für 7 Tage kultiviert. Dann wurde das Ausmaß der Rostpilzentwicklung auf den Blättern ermittelt.

Die visuell ermittelten Werte für den Prozentanteil befallener Blattflächen wurden in Wirkungsgrade als % der unbehandelten Kontrolle umgerechnet. Wirkungsgrad 0 ist gleicher Befall wie in der unbehandelten Kontrolle, Wirkungsgrad 100 ist 0 % Befall. Die zu erwartenden Wirkungsgrade für Wirkstoffkombinationen wurden nach der obengenannten Colby-Formel ermittelt und mit den beobachteten Wirkungsgraden verglichen.

Tabelle 3

| Wirkstoff | Wirkstoffkonzentration in der Spritzbrühe in ppm | Wirkungsgrad in % der unbehandelten Kontrolle |
|---|---|---|
| Kontrolle (unbehandelt) | (90 % Befall) | 0 |
| Verbindung I = Prothioconazol | 1<br>0.25<br>0,015<br>0,006 | 0<br>0<br>0<br>0 |
| Verbindung IV = Pyraclostrobin | 0.25<br>0,06 | 0<br>0 |

Tabelle 4

| Erfindungsgemäße Kombinationen | Beobachteter Wirkungsgrad | Berechneter Wirkungsgrad*) |
|---|---|---|
| Verbindung I = Prothioconazol + Verbindung IV = Pyraclostrobin 0,015+0,25 ppm Mischung 1 : 16 | 94 | 0 |
| Verbindung I = Prothioconazol + Verbindung IV = Pyraclostrobin 0,06+0,25 ppm Mischung 1 : 4 | 89 | 0 |

(fortgesetzt)

| Erfindungsgemäße Kombinationen | Beobachteter Wirkungsgrad | Berechneter Wirkungsgrad*) |
|---|---|---|
| Verbindung I = Prothioconazol + Verbindung IV = | | |
| Pyraclostrobin 1 + 0,25 ppm Mischung 4 : 1 | 22 | 0 |
| Verbindung I = Prothioconazol + Verbindung IV = Pyraclostrobin 0,25+0,06 ppm Mischung 4 : 1 | 22 | 0 |
| Verbindung I = Prothioconazol + Verbindung IV = Pyraclostrobin 1 + 0,06 ppm Mischung 16 : 1 | 89 | 0 |
| *) berechnet nach der Colby-Formel | | |

Aus den Ergebnissen des Versuches geht hervor, daß der beobachteter Wirkungsgrad in allen Mischungsverhältnissen höher ist, als nach der Colby-Formel vorausberechnete Wirkungsgrad (aus Synerg 171. XLS).

## Patentansprüche

**1.** Fungizide Mischung, enthaltend

(1) 2-[2-(1-Chlorcyclopropyl)-3-(2-chlorphenyl)-2-hydroxypropyl]-2,4-dihydro-[1,2,4]-triazol-3-thion (Prothioconazole) der Formel I oder dessen Salze oder Addukte

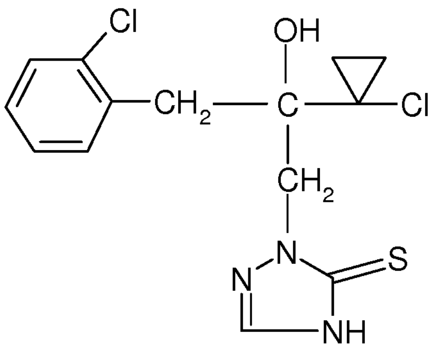

(I)

und
(4) Pyraclostrobin der Formel IV

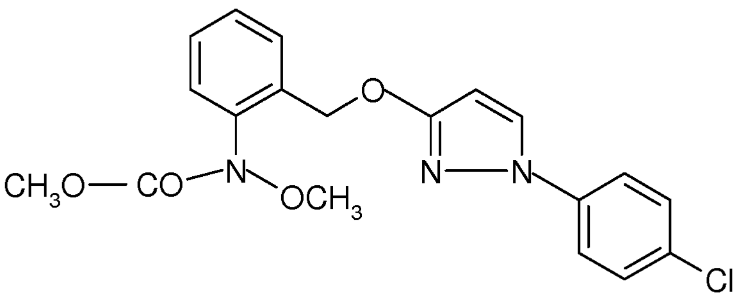

(IV) (Pyraclostrobin)

oder deren Salze oder Addukte in einer synergistich wirksamen Menge, wobei das Gewichtsverhältnis von Prothioconazole der Formel I zu Pyraclostrobin der Formel IV 20:1 bis 1:20 beträgt.

**2.** Verfahren zur Bekämpfung von Schadpilzen, **dadurch gekennzeichnet, daß** man die Schadpilze, deren Lebensraum oder die von ihnen freizuhaltenden Pflanzen, Samen, Böden, Flächen, Materialien oder Räume mit der fungiziden Mischung gemäß Anspruch 1 behandelt.

3. Verfahren nach Anspruch 2, **dadurch gekennzeichnet, daß** man die Verbindung der Formel I gemäß Anspruch 1 und der Verbindung der Formel IV gemäß Anspruch 1 gleichzeitig, und zwar gemeinsam oder getrennt, oder nacheinander ausbringt.

4. Verfahren nach Anspruch 2 oder 3, **dadurch gekennzeichnet, daß** man die fungizide Mischung oder die Verbindung der Formel I mit der Verbindung der Formel IV gemäß Anspruch 1 in einer Menge von 0,01 bis 8 kg/ha aufwendet.

5. Fungizide Mittel, enthaltend die fungizide Mischung gemäß Anspruch 1 sowie einen festen oder flüssigen Träger.

## Claims

1. A fungicidal mixture, comprising

(1) 2-[2-(1-chlorocyclopropyl)-3-(2-chlorophenyl) -2-hydroxypropyl]-2,4-dihydro-[1,2,4]-triazole-3-thione (prothioconazole) of the formula I or its salts or adducts

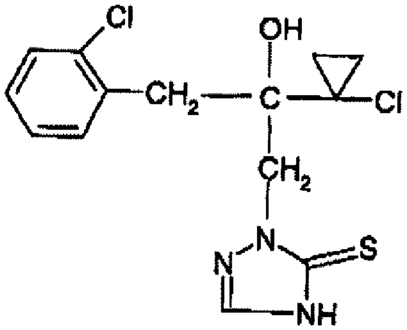

(I)

and
(4) pyraclostrobin of the formula IV

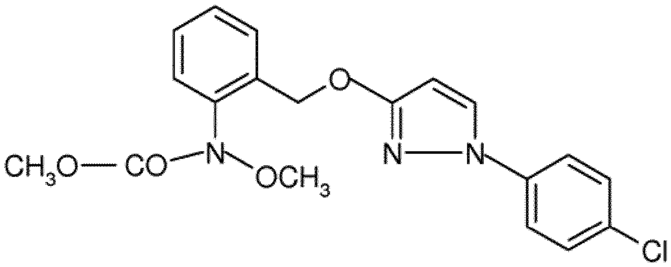

(IV) (Pyraclostrobin)

or its salts or adducts in a synergistically effective amount, wherein the weight ratio of prothioconazole of the formula I to pyraclostrobin of the formula IV is from 20:1 to 1:20.

2. A method for controlling harmful fungi, which comprises treating the harmful fungi, their habitat or the plants, seeds, soils, areas, materials or spaces to be kept free from them with the fungicidal mixture according to claim 1.

3. The method according to claim 2, wherein the compound of the formula I as set forth in claim 1 and the compound of the formula IV as set forth in claim 1 are applied simultaneously, that is together or separately, or in succession.

4. The method according to claim 2 or 3, wherein the fungicidal mixture or the compound of the formula I with the compound of the formula IV as set forth in claim 1 is applied in an amount of from 0.01 to 8 kg/ha.

5. A fungicidal composition, comprising the fungicidal mixture according to claim 1 and a solid or liquid carrier.

**Revendications**

1. Mélange fongicide, contenant :

   (1) la 2-[2-(1-chlorocyclopropyl)-3-(2-chlorophényl)-2-hydroxypropyl]-2,4-dihydro-[1,2,4]-triazol-3-thione (prothioconazole) de formule I ou ses sels ou ses produits d'addition :

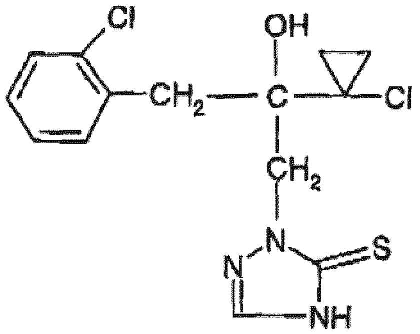

(I)

   et
   (4) la pyraclostrobine de formule IV :

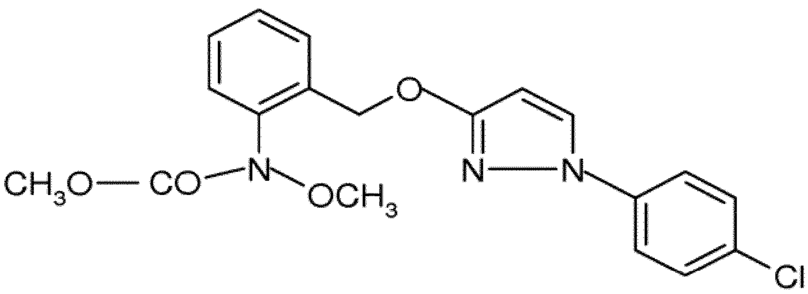

(IV) (pyraclostrobine)

   ou ses sels ou produits d'addition en quantité efficace au plan synergique, le rapport pondéral du prothioconazole de formule I à la pyraclostrobine de formule IV étant de 20:1 à 1:20.

2. Procédé pour lutter contre des champignons nuisibles, **caractérisé en ce que** l'on traite les champignons nuisibles, leur biotope ou les plantes, semences, sols, surfaces, matériaux ou espaces qui doivent en être débarrassés, avec le mélange fongicide selon la revendication 1.

3. Procédé selon la revendication 2, **caractérisé en ce que** l'on épand le composé de formule I selon la revendication 1 et le composé de formule IV selon la revendication 1 simultanément, à savoir conjointement ou séparément, ou successivement.

4. Procédé selon la revendication 2 ou 3, **caractérisé en ce que** l'on applique le mélange fongicide ou le composé de formule I avec le composé de formule IV selon la revendication 1, en quantité de 0,01 à 8 kg/ha.

5. Produits fongicides, contenant le mélange fongicide selon la revendication 1, ainsi qu'une substance véhiculaire solide ou liquide.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9616048 A
- WO 9847367 A
- EP 0804421 A


### In der Beschreibung aufgeführte Nicht-Patentliteratur


- **R.S. COLBY.** *Weeds,* 1967, vol. 15, 20-22